(19) Europäisches Patentamt
European Patent Office
Office européen des brevets

(11) **EP 2 924 416 A1**

(12) **EUROPÄISCHE PATENTANMELDUNG**

(43) Veröffentlichungstag:
**30.09.2015  Patentblatt 2015/40**

(51) Int Cl.:
***G01N 3/12*** *(2006.01)*  ***G01N 7/14*** *(2006.01)*
***G01N 33/28*** *(2006.01)*

(21) Anmeldenummer: **15158007.3**

(22) Anmeldetag: **06.03.2015**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA**

(30) Priorität: **12.03.2014  DE 102014204543**

(71) Anmelder: **SKF Lubrication Systems Germany GmbH**
**69190 Walldorf (DE)**

(72) Erfinder:
• **Schuermann, Stefan**
  **69190 Walldorf (DE)**
• **Labus, Matthias**
  **69231 Rauenberg-Rotenberg (DE)**
• **Teupner, Martin**
  **68809 Neulußheim (DE)**

(74) Vertreter: **Kuhstrebe, Jochen**
**SKF GmbH**
**IA&IP**
**Gunnar-Wester-Straße 12**
**97421 Schweinfurt (DE)**

(54)  **Verfahren und Vorrichtung zur Bestimmung des reinen Schmiermittelanteils eines aus einer Lageranordnung entnommenen Schmiermittels**

(57)     Verfahren zur Bestimmung des reinen Schmiermittelanteils eines aus einer Lageranordnung (1) entnommenen Schmiermittels (2). Um den reinen Schmiermittelanteil auch bei mit Gas verunreinigtem Schmiermittel genau erfassen zu können, sieht die Erfindung die Schritte vor: a) Fördern des zu entnehmenden Schmiermittels (2) von der Lageranordnung (1) in ein Messgefäß (3) mit definiertem Volumen, wobei mittels eines Förderelements (4) Schmiermittel (2) über eine Zeit oder in einer Anzahl portionierter Teilvolumina von der Lageranordnung (1) in das Messgefäß (3) gefördert wird; b) Messen des Drucks des Schmiermittels (2) im Messgefäß (3); c) Bestimmung der Zeit oder der Anzahl geförderter Teilvolumina, bis das Schmiermittel (2) im Messgefäß (3) einen vorgegebenen Druck aufweist; d) Bestimmung des prozentualen Schmiermittelanteils in einem geförderten Teilvolumen anhand eines gespeicherten funktionalen Zusammenhangs zwischen der Zeit oder der Anzahl portionierter Teilvolumina bis zum Erreichen des vorgegebenen Drucks im Messgefäß (3) und dem prozentualen Schmiermittelanteil.

Fig. 2

**EP 2 924 416 A1**

**Beschreibung**

Die Erfindung betrifft ein Verfahren sowie eine Vorrichtung zur Bestimmung des reinen Schmiermittelanteils eines aus einer Lageranordnung entnommenen gasförmig verunreinigten Schmiermittels.

Bei einer Lageranordnung, insbesondere bei einem Großlager, ist es ein wichtiger Parameter zum Erhalt der Leistungsfähigkeit der Lagerung, dass eine möglichst genaue Menge, d. h. Volumen, an Schmiermittel (insbesondere Schmierfett) im Lager ist, um einerseits eine Mangelschmierung und andererseits eine Überschmierung zu verhindern.

Durch den bekannten Zusammenhang des Volumens und der Masse über die Dichte kann die Masse des Schmierstoffes, sofern jene gewünscht oder benötigt wird, berechnet werden.

Nicht überall kann zur Mengenbestimmung, d. h. Feststellung der vorhandenen Masse an Schmiermittel, auf ein System zum Wiegen des Mediums zurückgegriffen werden. In diesen Fällen kann die Menge an Schmiermittel allerdings näherungsweise durch eine Bestimmung des Volumens ermittelt werden.

Hierbei besteht jedoch folgende Schwierigkeit: Ist ein zu vermessendes (flüssiges oder zähfließendes) Medium gasförmig verunreinigt, so kann das Volumen nicht mehr hinreichend genau bei der Volumenmessung ermittelt werden. Das im Medium eingeschlossene Gas lässt es nicht mehr zu, vom Volumen zuverlässig auf die Masse zu schließen (die Dichte des reinen gasfreien Schmierfetts bei definierter Temperatur ist an sich ja bekannt).

Wenngleich das Wiegen eine relativ einfache Möglichkeit ist, die Masse eines Mediums zu bestimmen, ist diese Möglichkeit eingeschränkt, wenn entsprechende gasförmige Einschlüsse im Medium vorliegen. In Anwendungen, bei denen das Wiegen nur eingeschränkt oder gar nicht angewendet werden kann (beispielsweise bei der Blattlagerung in Windenergieanlagen), ist somit eine exakte Massenbestimmung des entnommenen Schmierfetts bisher kaum möglich, da die vorhandenen Instrumente zur Volumenbestimmung durch die gasförmigen Einschlüsse im Medium zu einer verfälschten Messung führen. Des Weiteren ist insbesondere bei zähflüssigen Medien ein "Entlüften" (Trennen des Mediums von den gasförmigen Verunreinigungen) des Mediums nur eingeschränkt möglich oder technisch sehr aufwendig.

Somit stellen eingeschlossene Gasvolumina im Medium ein Problem bei der Ermittlung der entnommenen Masse Schmierfett aus einem Lager dar.

Aus der DE 10 2004 038 801 A1 ist ein Verfahren zur Erkennung und Ermittlung des in Schmierfett eingeschlossenen Gasvolumens bekannt, wobei eine definierte Menge des Schmierfetts in einem Messzylinder über einen Kolben einem definierten Druck ausgesetzt wird und dabei aus der sich einstellenden Längsverschiebung des Kolbens bzw. der Kolbenstange das im Schmierfett eingeschlossenen Gasvolumens ermittelt wird. Die EP 0 451 752 A2 offenbart ein Verfahren zur Ermittlung des in einer Flüssigkeit enthaltenen Gasanteils, wobei ein Flüssigkeitsvolumen in einem Messzylinder ausgehend von einem bekannten Druck durch Druckerhöhung auf ein Volumen komprimiert wird. Die DE 10 2010 032 010 A1 offenbart einen Sensor zur Erkennung von Gaseinschlüssen in einem Schmierfett.

Der Erfindung liegt die **Aufgabe** zugrunde, ein Verfahren der eingangs genannten Art sowie eine entsprechende Vorrichtung vorzuschlagen, mit dem bzw. mit der es möglich ist, den reinen Schmiermittelanteil des aus einem Lager entnommenen Schmiermittels möglichst genau zu bestimmen, auch wenn das Schmiermittel mit Gasen verunreinigt ist. Dadurch soll es möglich sein, auch das Volumen bzw. die Masse des Schmiermittels möglichst genau zu bestimmen, um das Nachschmieren des Lagers möglichst präzise vornehmen zu können.

Die **Lösung** dieser Aufgabe durch die Erfindung ist dadurch gekennzeichnet, dass das Verfahren die folgenden Schritte umfasst:

a) Fördern des zu entnehmenden Schmiermittels von der Lageranordnung in ein Messgefäß mit definiertem Volumen, wobei mittels eines Förderelements Schmiermittel über eine Zeit oder in einer Anzahl portionierter Teilvolumina von der Lageranordnung in das Messgefäß gefördert wird;

b) Messen des Drucks des Schmiermittels im Messgefäß;

c) Bestimmung der Zeit oder der Anzahl geförderter Teilvolumina, bis das Schmiermittel im Messgefäß einen vorgegebenen Druck aufweist;

d) Bestimmung des prozentualen Schmiermittelanteils in einem geförderten Teilvolumen anhand eines gespeicherten funktionalen Zusammenhangs zwischen der Zeit oder der Anzahl portionierter Teilvolumina bis zum Erreichen des vorgegebenen Drucks im Messgefäß und dem prozentualen Schmiermittelanteil.

Bevorzugt erfolgt im Anschluss an den genannten Schritt d) eine Bestimmung der Masse des Schmiermittels anhand des über der Zeit oder durch die Anzahl portionierter Teilvolumina vom Förderelement geförderten Volumens, der Dichte des Schmiermittels und des nach obigem Schritt d) bestimmten prozentualen Schmiermittelanteils. Die Bestimmung der Masse kann über das gemessene und in das Messgefäß geförderte Volumen an Schmiermittel und über den ermittelten prozentualen Schmiermittelanteil (bei angenommener Masselosigkeit des im Schmiermittel vorhandenen Gases) und unter Zugrundelegung der an sich bekannten Dichte des gasfreien Schmiermittels einfach erfolgen.

Der nach obigem Schritt c) vorgegebene Druck liegt dabei bevorzugt zwischen 2 bar und 6 bar, besonders bevorzugt zwischen 3 bar und 5 bar.

Das Fördern von zu entnehmendem Schmiermittel von der Lageranordnung in das Messgefäß erfolgt bevorzugt, indem ein Förderelement in Form einer Kolbenpumpe verwendet wird, das eine Anzahl portionierter Teilvolumina an Schmiermittel fördert.

Nach der Bestimmung der Zeit oder der Anzahl geförderter Teilvolumina nach obigem Schritt c) kann das Messgefäß geleert und der Prozess nach den obigen Schritten a) bis e) erneut durchgeführt werden. Hierdurch ist praktisch eine kontinuierliche bzw. stetige Bestimmung des reinen Schmiermittelanteils im entnommenen Schmiermittel möglich.

Hierbei ist bevorzugt vorgesehen, dass das Leeren des Messgefäßes nach Umsteuern einer Ventilanordnung erfolgt.

Eine Weiterbildung des erfindungsgemäßen Verfahrens sieht vor, dass die Bestimmung der Zeit oder der Anzahl geförderter Teilvolumina nach obigem Schritt c) von einem im Messgefäß herrschenden Referenzdruck aus erfolgt. Dieser Referenzdruck beträgt bevorzugt 1 bar.

Die Vorrichtung zur Bestimmung des reinen Schmiermittelanteils eines aus einer Lageranordnung entnommenen Schmiermittels weist erfindungsgemäß auf:

- ein Messgefäß mit definiertem Volumen zur Aufnahme von zu entnehmendem Schmiermittel,

- ein Förderelement zum Absaugen von Schmiermittel aus der Lageranordnung und Fördern desselben in das Messgefäß,

- Druckmessmittel zur Messung des Drucks im Messgefäß,

- eine Steuerungseinrichtung zur Registrierung des Drucks im Messgefäß, zum Vergleichen des Drucks mit einem vorgegebenen Wert und zur Ermittlung des prozentualen Schmiermittelanteils im aus der Lageranordnung abgesaugten Volumen an Schmiermittel anhand eines gespeicherten funktionalen Zusammenhangs zwischen der Zeit der Absaugung oder der Anzahl portionierter abgesaugter Teilvolumina bis zum Erreichen des vorgegebenen Drucks im Messgefäß und dem prozentualen Schmiermittelanteil.

Das Förderelement ist bevorzugt eine Kolbenpumpe.

Weiterhin kann eine Ventilanordnung vorgesehen sein, mit der wahlweise abzusaugendes Schmiermittel in das Messgefäß gefördert oder aus diesem in einen Sammelbehälter ausgebracht werden kann. Die Ventilanordnung ist bevorzugt ein Wegeventil.

Die Erfindung erlaubt somit die genaue Bestimmung des reinen Schmiermittelanteils des Schmiermittel-Luft-Gemisches, das aus der Lageranordnung entnommen wird, und damit auch eine präzise Volumenbestimmung des gasförmig verunreinigten Schmiermittels. D. h. das beschriebene Verfahren eignet sich zur Mengenbestimmung von fließenden und/oder zähfließenden Stoffen (beispielsweise Fett der NLGI Klasse 2), insbesondere, wenn der Stoff mit einem gasförmigen Medium (beispielsweise Luft) verunreinigt ist.

Aufgrund der hohen Kompressibilitätsdifferenz zwischen dem gasförmigen Stoff und dem fließenden und/oder zähfließenden Stoff können unter Einsatz eines Messgefäßes und durch Messung des Druckes im Gefäß Rückschlüsse auf das tatsächliche Volumen der beiden Stoffe gezogen werden.

Die Erfindung kommt vor allem bei Großlageranwendungen zum Einsatz, bei denen es zur Instandhaltung wichtig ist, dass sich eine genau vorgegebenes Volumen bzw. eine genau vorgegebene Masse an Schmierfett im Lager befindet. Hierbei ist insbesondere an Windenergieanlagen gedacht.

In vorteilhafter Weise arbeitet das vorgeschlagene Verfahren im gesamten Bandbereich von gasfreiem Schmiermittel bis zu vollständig mit Gas durchsetztem Schmiermittel.

Die Auflösung der Messung ist gut, da das Verhältnis der Kompressibilität zwischen Luft und Fett und abstrahiert zwischen gasförmigen und flüssigen Stoffen bei einem Faktor von ca. 10.000 liegt.

Die Einrichtung zur Ermittlung der Masse des Schmiermittels kann problemlos in sich bewegenden Anwendungen untergebracht werden.

Das Verfahren ist störungsanfällig gegenüber Vibrationen und Temperatureinflüssen.

Somit wird eine exakte Volumen- bzw. Massenbestimmung von gasförmig verunreinigten Fetten und Ölen ermöglicht.

In der Zeichnung ist ein Ausführungsbeispiel der Erfindung dargestellt. Es zeigen:

Fig. 1     schematisch eine Anordnung zur Ermittlung des Schmiermittelanteils bzw. des Volumens von Schmiermittel, das aus einer Lageranordnung abgesaugt wird,

Fig. 2     schematisch die Anordnung nach Fig. 1 mit weiteren dargestellten Komponenten und

Fig. 3     die Darstellung eines funktionellen Zusammenhangs zwischen der Anzahl der Hübe eines Förderelements und dem Fettanteil im geförderten Medium (d. h. Verlauf des prozentualen Fettanteils über der Anzahl der Hübe der Kolbenpumpe).

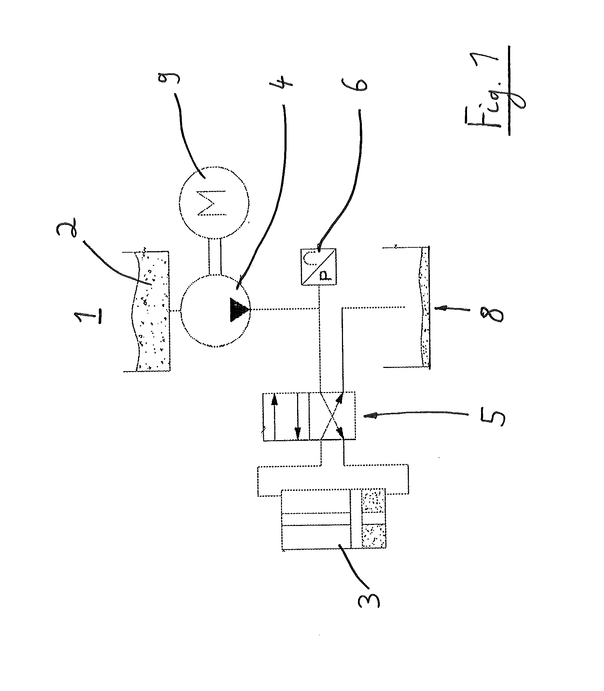

In Fig. 1 ist zunächst das Prinzip der Bestimmung des prozentualen Schmiermittelanteils bzw. der Volumenbestimmung von Schmiermittel 2 skizziert, das aus einer Lageranordnung 1 abgesaugt wird. Das Schmiermittel 2 wird von einem Förderelement 4 in Form einer Kolbenpumpe in ein Messgefäß 3 gefördert, das hier als Kolben-Zylinder-Element ausgebildet ist. Der Fluss des Schmiermittels 2 wird über eine Ventilanordnung 5 in Form eines Wegeventils gesteuert. Wesentlich sind noch Druckmessmittel 6, die den Druck im Messgefäß 3 erfassen.

Die Kolbenpumpe 4 wird von einem Motor 9 angetrieben. Bei jedem Hub der Kolbenpumpe 4 wird ein definiertes Volumen an Schmiermittel 2 in das Messgefäß 3 gefördert. Das Schmiermittel 2 ist allerdings mit Luft versetzt und insoweit verschmutzt. Dennoch kann das Volumen des Schmiermittels 2 ermittelt werden, so dass nach dem Absaugen von Schmiermittel 2 aus der Lageranordnung dasselbe Volumen ersetzt werden kann.

Es gilt folgendes:

Wird einem definierten Raum (d. h. einem definierten Volumen) kontinuierlich oder zyklisch ein bekanntes Volumen eines Mediums (reines Medium, gasförmig verunreinigtes Medium oder nur gasförmige Verunreinigung) zugeführt, so steigt der Druck in diesem System an. Der Druckanstieg erfolgt umso schneller, je geringer der Anteil an gasförmiger Verunreinigung im zugeführten Medium ist.

Dies kann in der Anordnung nach Fig. 1 zur Bestimmung des geförderten Mediumvolumens wie dargestellt umgesetzt werden.

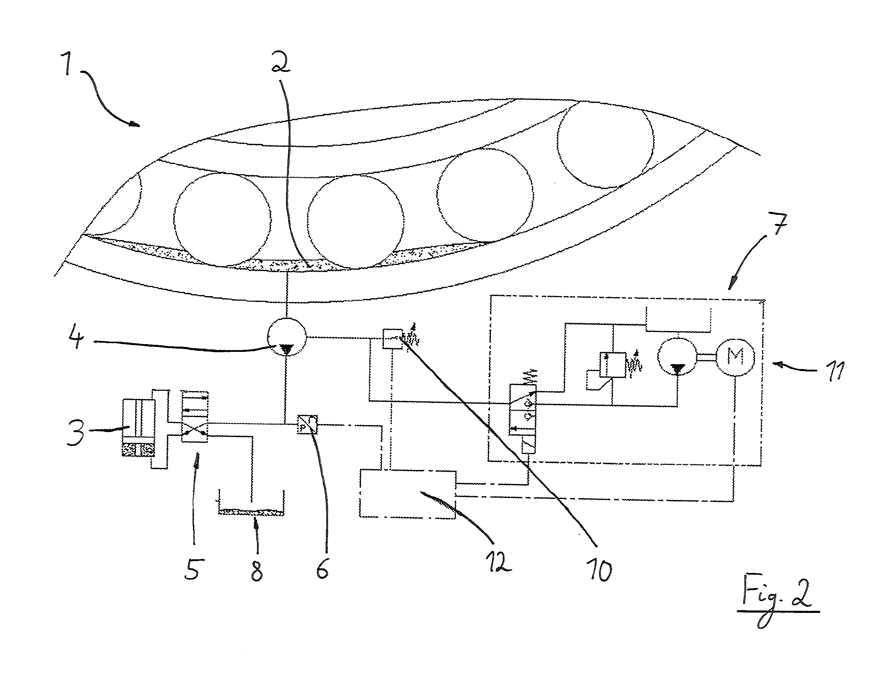

Die Kolbenpumpe 4 (mit beliebigem Antrieb) fördert das Gemisch (Schmiermittel plus gasförmige Verunreinigung) in das Messgefäß 3 mit definiertem Volumen. Mit zunehmender Zeit bzw. mit zunehmender Hubzahl der Kolbenpumpe 4, die von einem Zählwerk erfasst wird (nämlich von der Steuerungseinrichtung 7, s. Fig. 2), erhöht sich der Druck (überwacht durch das Druckmessmittel 6) im System in Abhängigkeit des Schmiermittelanteils im Gemisch bestehend aus Schmiermittel und eingeschlossener Luft. Bei Erreichen eines vorgegebenen Enddruckes schaltet das Wegeventil 5 die Leitungen um, und der Vorgang kann erneut starten. Das bereits bestimmte Volumen wird hierbei in den Sammelbehälter 8 entleert.

Aus der benötigten Zeit bzw. den benötigten Hüben zum Erreichen des genannten Enddruckes kann nun der Anteil an gefördertem Medium berechnet werden.

Die Kompressibilität von Flüssigkeiten, Festkörpern und Gasen berechnet sich nach der Gleichung

$$K = -\frac{dV}{V*d} \qquad\qquad (I)$$

Hierbei ist:

$\kappa$:     die Kompressibilität des Mediums,
V:     das Volumen des Mediums,
dp:     die Druckänderung auf das Volumen V und
dV     die Volumenänderung.

Wird einem Anfangsvolumen eine Druckänderung zugeführt, so ändert sich das resultierende Volumen proportional mit der Kompressibilität. Dieser Effekt wird vorliegend in abgewandelter Form zur Volumenbestimmung eines fließenden oder zähfließenden Mediums $V_F$ (hier Fett der Klasse NLGI 2) mit einer gasförmigen Verunreinigung mit dem Volumen $V_L$ genutzt.

Ein definiertes Anfangsvolumen V wird solange mit einem partiellen Volumen $dV_G$ beaufschlagt, bis ein vorgegebener Grenzdruck dp erreicht ist.

Aus der Gleichung (I) ergibt sich durch Umformung die Funktionsgleichung:

$$V_F = V - V_L, \text{ wobei } V_L(dV_L) = -\frac{dV_L}{K_L * d} \qquad (II)$$

Daraus resultiert:

$$V_F(dV_L) = V + \frac{dV_L}{K_L * d} \qquad (III)$$

Die Vernachlässigung der Kompressibilität von Fett ist zulässig, da unter Beaufschlagung eines für beiden Medien gleichen Druckes dp unter begrenztem Gesamtvolumen eines Gefäßes gilt:

$$V_L + V_F = -\frac{dV_L}{K_L * d} - \frac{dV_F}{K_F * d} \qquad (IV)$$

Durch den Unterschied zwischen festen oder flüssigen Stoffen (hier mit Index "F") und gasförmigen Stoffen (hier mit Index "L") mit einem Verhältnis von ca. 10.000 gilt:

$$K_L \gg K_F \qquad (V)$$

Daher ist die Annahme gerechtfertigt, dass feste, aber auch flüssige Stoffe in erster Näherung das Gesamtvolumen V begrenzen und schlussendlich im Vergleich zu gasförmigen Stoffen als praktisch nicht kompressibel angesehen werden können. Luft begrenzt das Gesamtvolumen zwar auch, aber um den Faktor 10.000 schwächer, da es deutlich kompressibler ist.

Dieser Umstand wird vorliegend genutzt. Über die Funktionsgleichung (II) lässt sich dann bei Festlegen eines Anfangs- und Grenzdruckes, also dp, und bei bekannter Zufuhr an dV (sei es zeitgesteuert oder hubgesteuert) ein eindeutiger Rückschluss auf die Anteile an festen/flüssigen oder gasförmigen Volumina treffen.

Demgemäß stellt die vorliegende Idee auf ein Verfahren und eine entsprechende Vorrichtung zur quasi-kontinuierlichen Bestimmung des Volumens flüssigen und/oder zähfließenden gasförmig verunreinigten Stoffen ab.

In Fig. 2 ist eine etwas konkretere Ausgestaltung der Vorrichtung zur Ermittlung der Masse des aus der Lageranordnung 1 entnommenen Schmiermittels 2 skizziert.

In der dargestellten Anwendung soll aus einem fettgeschmierten Großlager "verbrauchter" Schmierstoff 2 entfernt werden, um "unverbrauchten" Schmierstoff nachfüllen zu können.

Hierzu wird vom Förderelement 4, das als Absaugelement fungiert (z. B. ausgebildet als hydraulisch angetriebene Kolbenpumpe) alter Schmierstoff, aber gegebenenfalls auch Luft aus dem Lager entnommen bzw. abgesaugt. Dieses Gemisch wird in das Messgefäß 3 gefördert und dort mit zunehmender Zyklenzahl (Anzahl der Kolbenhübe der Kolbenpumpe 4) immer weiter verdichtet. Die Anzahl der Zyklen wird über den Antrieb der Kolbenpumpe 4 bestimmt und von der Steuerungseinrichtung 7 registriert. Dargestellt ist dabei der Antrieb 11 für das Förderelement 4 (mit elektrischem Antrieb, Druckbegrenzungsventil und Entlastungsventil) und die Steuerung 12 selber.

In der vorliegenden Anwendung wird hierfür die elektrisch angetriebene Kolbenpumpe 4 verwendet, die über ein 3-2-Wege-Ventil 5 abwechselnd den Betriebsdruck für das Absaugelement auf- und wieder abbaut. Dieser Druckverlauf wird von einem Druckschalter 10 überwacht, dessen Signal zusätzlich von der Steuerungseinrichtung 7 mitgezählt wird. Wird nun im Messgefäß 3 (Messzylinder) ein definierter Druck (im vorliegenden Ausführungsbeispiel: 3 bar) erreicht, schaltet die Ventilanordnung 5 ein Umsteuerventil, und ein neuer Messvorgang wird gestartet. Aus der Anzahl der für den Druckaufbau bis zum genannten Druck von 3 bar benötigten Zyklen wird das Volumen an gefördertem Altfett bestimmt und dieses während des nächsten Messzyklus in den Sammelbehälter 8 weitergefördert.

Alternativ zu diesem Vorgehen kann mit der Erfassung der benötigten Hübe der Kolbenpumpe 4 auch erst bei einem definierten Anfangsdruck (bevorzugt: 1 bar) begonnen werden. Hiermit werden die Hübe, die zum Umschieben des Kolbens im Messzylinder benötigt werden, für die Messung ausgeblendet.

Im Ausführungsbeispiel wurde hierzu eine Diagrammschar mit diversen prozentualen Anteilen an Fett gegenüber der Anzahl an Hüben der Kolbenpumpe erstellt. Hier wurde beispielhaft ein Grenzdruck von 3 bar und von 5 bar dargestellt.

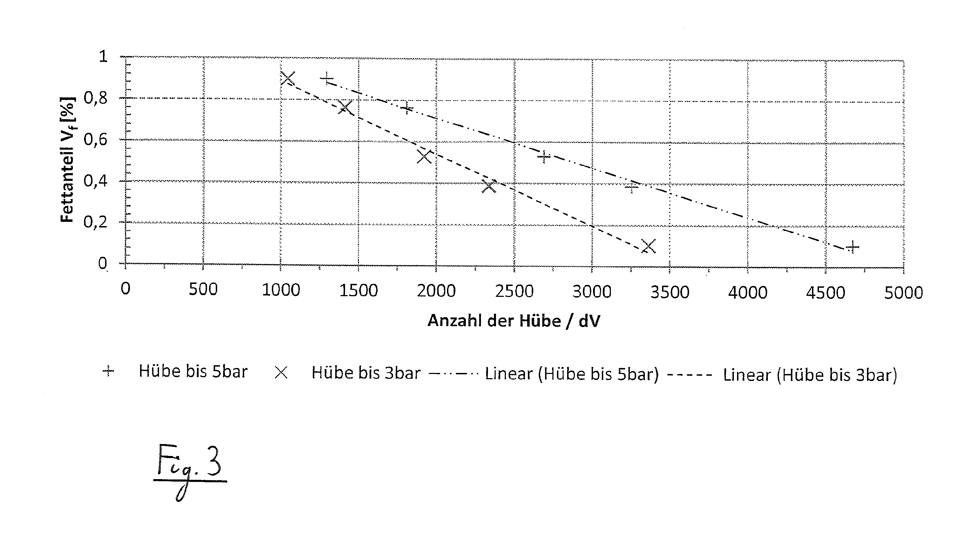

Der ermittelte Verlauf ist in Fig. 3 dargestellt.

Die Abszisse kann zeitdiskret oder kontinuierlich skaliert werden. Über den dargestellten funktionalen Verlauf ergibt sich die Ermittlung des Fett- bzw. Gasanteiles (angegeben in %) bei bekanntem Volumen des Messzylinders und des Grenzdruckes (in Fig. 3: dargestellt für 3 bar und für 5 bar) aus der Anzahl der Hübe der Kolbenpumpe 4 bzw. der Zeit deren Betrieb.

Fig. 3 zeigt also das mathematische Abbild des Fettanteils über der Anzahl der Hübe der Kolbenpumpe 4 bei zwei verschiedenen Enddrücken von 3 bzw. von 5 bar.

Die Anzahl der Hübe beschreibt die Gesamtanzahl der Zyklen der Kolbenpumpe von dem Status, bei dem das Messgefäß 3 leer ist, bis der gegebene (End)Druck erreicht ist.

In der Darstellung gemäß Fig. 3 wird ein Messversuchsdiagramm des Anteils an Fett über den partiellen Volumenanteilen (hier: Anzahl der Hübe der Kolbenpumpe 4) dargestellt. Vergleicht man die Messkurve mit der Herleitung gemäß obiger Gleichung (III) wird ersichtlich, dass die negative Steigung durch die Druckdifferenz entsteht, denn außerhalb des Messgefäßes 3 wird das Medium mit praktisch Null bar Überdruck in das Messgefäß mit Überdruck befördert. In praktischen Versuchen haben sich 3 bar bzw. 5 bar als besonders bevorzugt gezeigt. Somit beziffert sich dp auf 0 bar - 3 bar = - 3 bar bzw. 0 bar - 5 bar = - 5 bar.

**Bezugszeichenliste**

| 1 | Lageranordnung |
|---|---|
| 2 | Schmiermittel |
| 3 | Messgefäß |
| 4 | Förderelement (Kolbenpumpe; Absaugelement) |
| 5 | Ventilanordnung (Wegeventil) |
| 6 | Druckmessmittel |
| 7 | Steuerungseinrichtung |
| 8 | Sammelbehälter |
| 9 | Motor |
| 10 | Druckschalter |
| 11 | Antrieb für Förderelement |
| 12 | Steuerung |

**Patentansprüche**

1. Verfahren zur Bestimmung des reinen Schmiermittelanteils eines aus einer Lageranordnung (1) entnommenen Schmiermittels (2), umfassend die Schritte:

   a) Fördern des zu entnehmenden Schmiermittels (2) von der Lageranordnung (1) in ein Messgefäß (3) mit definiertem Volumen, wobei mittels eines Förderelements (4) Schmiermittel (2) über eine Zeit oder in einer Anzahl portionierter Teilvolumina von der Lageranordnung (1) in das Messgefäß (3) gefördert wird;
   b) Messen des Drucks des Schmiermittels (2) im Messgefäß (3);
   c) Bestimmung der Zeit oder der Anzahl geförderter Teilvolumina, bis das Schmiermittel (2) im Messgefäß (3) einen vorgegebenen Druck aufweist;
   d) Bestimmung des prozentualen Schmiermittelanteils in einem geförderten Teilvolumen anhand eines gespeicherten funktionalen Zusammenhangs zwischen der Zeit oder der Anzahl portionierter Teilvolumina bis zum Erreichen des vorgegebenen Drucks im Messgefäß (3) und dem prozentualen Schmiermittelanteil.

2. Verfahren nach Anspruch 1, **dadurch gekennzeichnet, dass** im Anschluss an Schritt d) von Anspruch 1 eine Bestimmung der Masse des Schmiermittels (2) erfolgt anhand des über der Zeit oder durch die Anzahl portionierter Teilvolumina vom Förderelement (4) geförderten Volumens, der Dichte des Schmiermittels (2) und des nach Schritt d) von Anspruch 1 bestimmten prozentualen Schmiermittelanteils.

3. Verfahren nach Anspruch 1 oder 2, **dadurch gekennzeichnet, dass** der nach Schritt c) gemäß Anspruch 1 vorgegebene Druck zwischen 2 bar und 6 bar liegt, vorzugsweise zwischen 3 bar und 5 bar.

**4.** Verfahren nach einem der Ansprüche 1 bis 3, **dadurch gekennzeichnet, dass** das Fördern von zu entnehmendem Schmiermittel (2) von der Lageranordnung (1) in das Messgefäß (3) erfolgt, indem ein Förderelement (4) in Form einer Kolbenpumpe verwendet wird, das eine Anzahl portionierter Teilvolumina an Schmiermittel (2) fördert.

**5.** Verfahren nach einem der Ansprüche 1 bis 4, **dadurch gekennzeichnet, dass** nach der Bestimmung der Zeit oder der Anzahl geförderter Teilvolumina nach Schritt c) gemäß Anspruch 1 das Messgefäß (3) geleert und der Prozess nach den Schritten a) bis d) gemäß Anspruch 1 erneut durchgeführt wird.

**6.** Verfahren nach Anspruch 5, **dadurch gekennzeichnet, dass** das Leeren des Messgefäßes (3) nach Umsteuern einer Ventilanordnung (5) erfolgt.

**7.** Verfahren nach einem der Ansprüche 1 bis 6, **dadurch gekennzeichnet, dass** die Bestimmung der Zeit oder der Anzahl geförderter Teilvolumina nach Schritt c) gemäß Anspruch 1 von einem im Messgefäß (3) herrschenden Referenzdruck aus erfolgt, wobei der Referenzdruck insbesondere 1 bar beträgt.

**8.** Vorrichtung zur Bestimmung des reinen Schmiermittelanteils eines aus einer Lageranordnung (1) entnommenen Schmiermittels (2), zur Durchführung des Verfahrens nach einem der Ansprüche 1 bis 7, wobei die Vorrichtung aufweist:

- ein Messgefäß (3) mit definiertem Volumen zur Aufnahme von zu entnehmendem Schmiermittel (2),
- ein Förderelement (4) zum Absaugen von Schmiermittel (2) aus der Lageranordnung (1) und Fördern desselben in das Messgefäß (3),
- Druckmessmittel (6) zur Messung des Drucks im Messgefäß (3),
- eine Steuerungseinrichtung (7) zur Registrierung des Drucks im Messgefäß (3), zum Vergleichen des Drucks mit einem vorgegebenen Wert und zur Ermittlung des prozentualen Schmiermittelanteils im aus der Lageranordnung (1) abgesaugten Volumen an Schmiermittel (2) anhand eines gespeicherten funktionalen Zusammenhangs zwischen der Zeit der Absaugung oder der Anzahl portionierter abgesaugter Teilvolumina bis zum Erreichen des vorgegebenen Drucks im Messgefäß (3) und dem prozentualen Schmiermittelanteil.

**9.** Vorrichtung nach Anspruch 8, **dadurch gekennzeichnet, dass** das Förderelement (4) eine Kolbenpumpe ist.

**10.** Vorrichtung nach Anspruch 8 oder 9, **dadurch gekennzeichnet, dass** eine Ventilanordnung (5) vorgesehen ist, mit der wahlweise abzusaugendes Schmiermittel (2) in das Messgefäß (3) gefördert oder aus diesem in einen Sammelbehälter (8) ausgebracht werden kann.

**11.** Vorrichtung nach Anspruch 10, **dadurch gekennzeichnet, dass** die Ventilanordnung (5) ein Wegeventil ist.

Fig. 1

Fig. 2

Fig. 3

Europäisches
Patentamt

European
Patent Office

Office européen
des brevets

**EUROPÄISCHER RECHERCHENBERICHT**

Nummer der Anmeldung

EP 15 15 8007

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X,D | DE 10 2004 038801 A1 (BAYERISCHE MOTOREN WERKE AG [DE]; PROWIN GMBH [DE]) 23. Februar 2006 (2006-02-23) | 1,4,8-11 | INV. G01N3/12 G01N7/14 G01N33/28 |
| A | * Absätze [0001], [0012], [0013], [0014]; Abbildung 1 * ----- | 2,3,5-7 | |
| A | CN 1 296 694 C (UNIV SHANGHAI COMMUNICATION [CN]) 24. Januar 2007 (2007-01-24) * Abbildung 1 * ----- | 1,8 | |
| A | US 4 164 137 A (WILLIAMSON WILLIAM A [US]) 14. August 1979 (1979-08-14) * Abbildung 1 * * Spalte 1, Zeile 64 - Spalte 2, Zeile 25 * * Spalte 2, Zeile 51 - Zeile 63 * ----- | 1,8 | |
| A | FR 2 858 056 A1 (RENAULT SA [FR]) 28. Januar 2005 (2005-01-28) * Zusammenfassung; Abbildung 2 * ----- | 1,8 | |

RECHERCHIERTE
SACHGEBIETE (IPC)

G01N

Der vorliegende Recherchenbericht wurde für alle Patentansprüche erstellt

| Recherchenort | Abschlußdatum der Recherche | Prüfer |
|---|---|---|
| Den Haag | 24. August 2015 | Bockstahl, Frédéric |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument
...............................................................................................
& : Mitglied der gleichen Patentfamilie, übereinstimmendes
    Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 15 15 8007

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

24-08-2015

| Im Recherchenbericht angeführtes Patentdokument | Datum der Veröffentlichung | Mitglied(er) der Patentfamilie | Datum der Veröffentlichung |
|---|---|---|---|
| DE 102004038801 A1 | 23-02-2006 | KEINE | |
| CN 1296694 C | 24-01-2007 | KEINE | |
| US 4164137 A | 14-08-1979 | KEINE | |
| FR 2858056 A1 | 28-01-2005 | KEINE | |

EPO FORM P0461

Für nähere Einzelheiten zu diesem Anhang : siehe Amtsblatt des Europäischen Patentamts, Nr.12/82

**EP 2 924 416 A1**

**IN DER BESCHREIBUNG AUFGEFÜHRTE DOKUMENTE**

**In der Beschreibung aufgeführte Patentdokumente**

- DE 102004038801 A1
- EP 0451752 A2
- DE 102010032010 A1